# EUROPÄISCHE PATENTANMELDUNG

(11) **EP 2 821 042 A1**
(43) Veröffentlichungstag der Anmeldung: **07.01.2015**
(21) Anmeldenummer: 13174644.8
(22) Anmeldetag: 02.07.2013
(51) Int. Cl.: A61F 13/20, A61F 13/26

(54) **Tamponapplikator und Verfahren zur Montage**

(71) Anmelder: Ruggli Projects AG, 6332 Hagendorn (CH)
(72) Erfinder: Rolli, Kilian, CH-5400 Baden (CH); Hammen, Axel, 5426 Lengnau (CH)
(74) Vertreter: AMMANN PATENTANWÄLTE AG BERN

(57) **Zusammenfassung**

Der Tamponapplikator (2) besteht aus einer Hülse (3) zur Aufnahme eines Tampons (5) und einem Stössel (4) zum Ausstossen des Tampons (5) aus der Hülse (3). Die Hülse (3) hat ein Einführungsende (7) und ein stösselseitiges Ende (11) sowie im Bereich des stösselseitigen Endes einen Führungsbereich für den Stössel (4). Der Stössel (4) hat ein erstes, aufgeweitetes Ende (12) für den Kontakt mit dem Tampon (5) und ein zweites, aufgeweitetes Ende (13) für den Kontakt mit einem Finger, wobei der Innendurchmesser im Führungsbereich der Hülse (3) kleiner ist als der Aussendurchmesser des ersten und zweiten Endes (12, 13) des Stössels (4). Um die Montage des Stössels (4) zu ermöglichen, ist der Führungsbereich durch eine Anzahl am Innenumfang der Hülse (3) verteilt angeordnete, radial nachgiebige Federlamellen (14) gebildet.

## Beschreibung

Die Erfindung betrifft einen Tamponapplikator mit einer Hülse zur Aufnahme eines Tampons und einem Stössel zum Ausstossen des Tampons aus der Hülse, wobei die Hülse ein Einführungsende und ein stösselseitiges Ende sowie im Bereich des stösselseitigen Endes einen Führungsbereich für den Stössel hat, wobei der Stössel ein erstes, aufgeweitetes Ende für den Kontakt mit dem Tampon und ein zweites, aufgeweitetes Ende für den Kontakt mit einem Finger hat und wobei der Innendurchmesser im Führungsbereich der Hülse kleiner ist als der Aussendurchmesser des ersten und zweiten Endes des Stössels.

Derartige Tamponapplikatoren sind bekannt. Die aufgeweiteten Enden des Stössels bewirken dabei nicht nur einen vergrösserten Kontaktbereich für den Tampon bzw. den Finger, sondern verhindern auch das Herausfallen des Stössels aus der Hülse. Für das Einbringen des Stössels in die Hülse sind bisher zwei Methoden bekanntgeworden. Gemäss dem Patent US7044928B2 wird der Stössel zunächst als Hohlzylinder hergestellt und in den Führungsbereich der Hülse eingeführt, wonach die Enden des Stössels durch ein erhitztes Werkzeug aufgeweitet werden. Nach der Patentanmeldung WO2012107880A2 ist der Führungsbereich durch eine Mehrzahl von Rippen gebildet, die sich im stösselseitigen Ende der Hülse radial nach innen erstrecken und der Stössel wird mit einem aufgeweiteten Ende in den Führungsbereich gedrückt, derart, dass sich die Aufweitung während der Bewegung durch den Führungsbereich deformiert und nach dem Verlassen des Führungsbereichs wiederhergestellt wird.

Es ist offensichtlich, dass die Methode nach US7044928B2 verhältnismässig aufwendig ist, weil der zunächst zylindrische Stössel in die Hülse gebracht und danach beidseitig aufgeweitet werden muss. Die Methode gemäss WO2012107880A2 ist nur mit hohlen, relativ dünnwandigen Stösseln anwendbar, weil eines der aufgeweiteten Enden des Stössels durch die radialen Rippen im Führungsbereich verformbar sein muss. Nicht hohle und relativ dickwandige Stössel, wie auch Stössel, bei denen das durch den Führungsbereich zu bringende Ende eine Verdickung der Wandstärke aufweist, sind mit dieser Methode nicht montierbar. Eine Verdickung der Wandstärke kann beispielsweise durch thermisches Verformen eines zylindrischen Stösselendes oder durch Spritzgiessen des Stössels entstehen.

Ausgehend von diesem Stand der Technik liegt der Erfindung die Aufgabe zu Grunde, einen Tamponapplikator vorzuschlagen, bei dem der Stössel einfach montierbar ist, auch wenn dieser dickwandig ist oder mindestens ein in der Wandstärke verdicktes Ende aufweist.

Diese Aufgabe wird erfindungsgemäss dadurch gelöst, dass der Führungsbereich durch eine Anzahl am Innenumfang der Hülse verteilt angeordnete, radial nachgiebige Federlamellen gebildet ist.

Diese erfindungsgemässe Lösung hat insbesondere den Vorteil, dass die Federlamellen bei der Montage des Stössels elastisch nachgeben und daher an den Stössel keine besondere Anforderungen hinsichtlich seiner Elastizität gestellt werden müssen. Da ausserdem die Federlamellen radial nachgiebig sind, können sie so dimensioniert werden, dass sie spielfrei am zwischen den Enden des montierten Stössels liegenden Teil des Stössels anliegen und somit den Stössel optimal führen. Darüber hinaus können die Federlamellen den Stössel auf einem grossen Teil seines Umfangs führen, was bei den Rippen gemäss WO2012107880A2 nicht möglich ist.

Die im vorliegenden Zusammenhang verwendeten Begriffe Innendurchmesser und Aussendurchmesser sollen die Erfindung nicht auf Hülsen und Stössel mit kreisrundem Querschnitt beschränken, sondern auch andere wie beispielsweise ovale oder polygone Querschnittsformen umfassen.

Nach einer Ausführungsart der Erfindung hat die Hülse einen Aufnahmebereich für den Tampon und einen im Bereich des stösselseitigen Endes angeordneten Griffbereich, dessen Aussendurchmesser kleiner ist als der Aussendurchmesser des Aufnahmebereichs. Dadurch wird die Handhabung des Tamponapplikators verbessert.

Gemäss einer weiteren Ausführungsart ist der Stössel hohl und das erste Ende und/oder das zweite Ende des Stössels weist eine grössere Wandstärke auf als der zwischen den Enden liegende Teil des Stössels. Durch diese Ausbildung wird die Herstellung des Stössels vereinfacht, indem der Stössel beispielsweise durch Spritzgiessen hergestellt werden kann.

Eine andere Ausführungsart sieht vor, dass die Federlamellen sich vom Innenumfang der Hülse in Längsrichtung der Hülse erstrecken und zur Längsachse der Hülse hin geneigt sind. Durch diese Neigung wird das Einführen des vorangehenden Endes des Stössels bei der Stösselmontage erleichtert. Diese Neigung kann gemäss weiteren Ausführungsarten zum stösselseitigen Ende oder zum Einführungsende der Hülse gerichtet sein. Entsprechend kann der Stössel bei der Montage am Einführungsende oder am stösselseitigen Ende in die Hülse eingeführt werden.

Entsprechend einer weiteren Ausführungsart weisen die Federlamellen einen sich vom Innenumfang der Hülse im Querschnitt gesehen in Richtung einer Sehne erstreckenden ersten Teil und daran anschliessend einen sich im Wesentlichen parallel zum Innenumfang der Hülse erstreckenden zweiten Teil auf. Dabei erstreckt sich der Bereich, in dem die Federlamellen mit der Wand der Hülse verbunden ist, im Wesentlichen in Längsrichtung der Hülse. Diese Ausführungsart ist kombinierbar mit der vorangehend beschriebenen Ausführungsart, bei der die Federlamellen an einem ihrer Längsenden auf einem umlaufenden Teil mit der Hülse verbunden sind.

Nach einer weiteren Ausführungsart ist vorgesehen, dass die Federlamellen zwischen dem ersten Teil und dem zweiten Teil um mehr als 90 Grad umgebogen sind. Der umgebogene Bereich wirkt wie eine Biegefeder und die Federlamellen können im Umfangsrichtung kürzer gestaltet werden, was wiederum die Anordnung einer grösseren Anzahl von Federlamellen gestattet.

Gemäss einer weiteren Ausführungsart erstreckt sich der zweite Teil der Federlamellen in Umfangsrichtung auf beiden Seiten des ersten Teils. Dadurch kann der Umfang des Führungsbereichs nahezu lückenlos ausgebildet werden.

Ein weiterer Aspekt der Erfindung betrifft die Montage des erfindungsgemässen Tamponapplikators, die auch mit einem relativ starren, im Extremfall sogar einem nicht hohlen Stössel möglich sein soll. Dem entsprechend ist vorgesehen, dass der Stössel montiert wird, indem er von einem Ende der Hülse durch den Führungsbereich geschoben wird, dass dabei das vorangehende Ende des Stössels die Federlamellen in einer im Wesentlichen radialen Richtung nach aussen drängt, so dass der Durchmesser des Führungsbereichs vorübergehend aufgeweitet wird und dass nach dem Durchgang des vorangehenden Endes durch den Führungsbereich die Federlamellen ihre Position wieder einnehmen, die sie vor dem Durchgang des vorangehenden Endes des Stössels hatten.

Ausführungsbeispiele der Erfindung werden nachstehend unter Bezugnahme auf die angefügten Zeichnungen beispielsweise näher beschrieben. Es zeigt
- Figur 1: einen Längsschnitt einer Tampon-Applikator-Anordnung;
- Figur 2: einen Ausschnitt aus Figur 1 in einem gegenüber Figur 1 vergrösserten Massstab, ohne Tampon und Rückzugsfaden;
- Figur 3: eine perspektivische Ansicht der Hülse gemäss den Figuren 1 und 2;
- Figur 4: einen die Finger-Erweiterung des Stössels zeigenden Ausschnitt aus Figur 1 in einem gegenüber Figur 1 vergrösserten Massstab, ohne den Rückzugsfaden des Tampons;
- Figur 5: eine Ansicht vom stösselseitigen Ende her auf eine weitere Ausführungsart der Hülse des TamponApplikators;
- Figur 6: eine Ansicht vom stösselseitigen Ende her auf eine weitere Ausführungsart der Hülse des TamponApplikators und
- Figur 7: eine Ansicht vom stösselseitigen Ende her auf eine weitere Ausführungsart der Hülse des TamponApplikators.

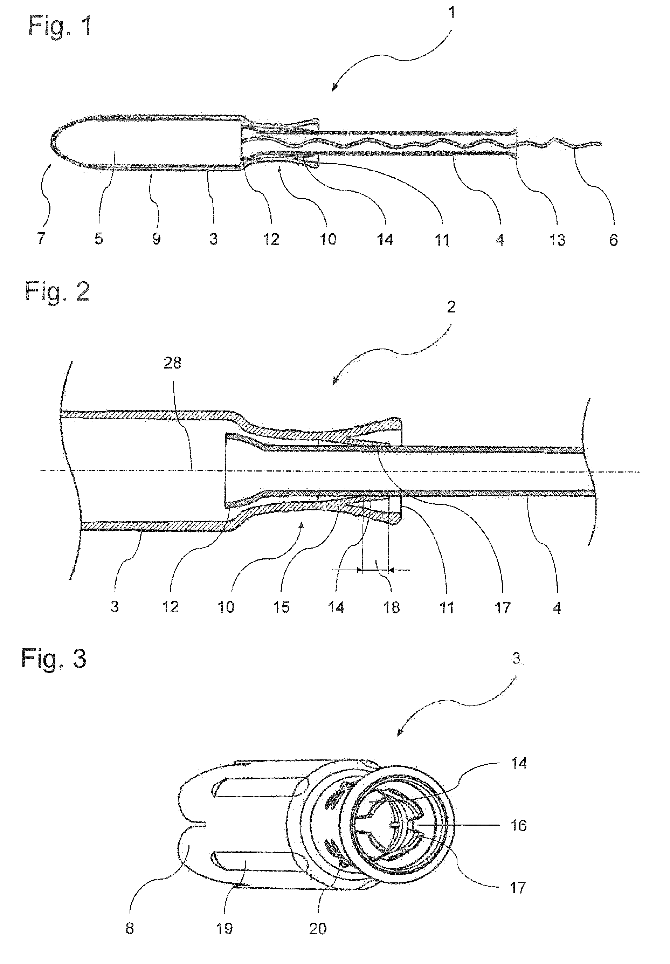

Die Figuren 1 bis 4 zeigen ein erstes Ausführungsbeispiel des erfindungsgemässen Tamponapplikators 2, wobei in Figur 1 zur Übersicht ein Längsschnitt einer Tampon-Applikator-Anordnung 1 dargestellt ist. Die Anordnung 1 besteht aus einer Hülse 3 mit einer Längsachse 28, einem in einem Aufnahmebereich 9 der Hülse 3 aufgenommenen Tampon 5 mit einem Rückzugsfaden 6 und einem Stössel 4 zum Ausstossen des Tampons 5 aus der Hülse 3. Die Hülse 3 und der Stössel 4 bilden den Tamponapplikator. Das Einführungsende 7 der Anordnung 1 ist in der Darstellung gemäss Figur 1 verschlossen, indem eine Anzahl von an der Hülse 3 angeformten Lippen 8 (siehe Figur 3) nach innen gebogen sind. In Figur 3, welche die Hülse 3 allein in einer perspektivischen Ansicht zeigt, sind die Lippen 8 offen dargestellt. An ihrem stösselseitigen Ende 11 hat die Hülse 3 einen gegenüber dem Aufnahmebereich 9 im Durchmesser reduzierten Griffbereich 10. Diese Durchmesserreduktion ist nicht zwingend, der Griffbereich 10 könnte auch den gleichen Aussendurchmesser haben wie der Aufnahmebereich 9. Der Stössel 4 hat an seinen Enden eine tamponseitige Erweiterung 12 und eine Finger-Erweiterung 13.

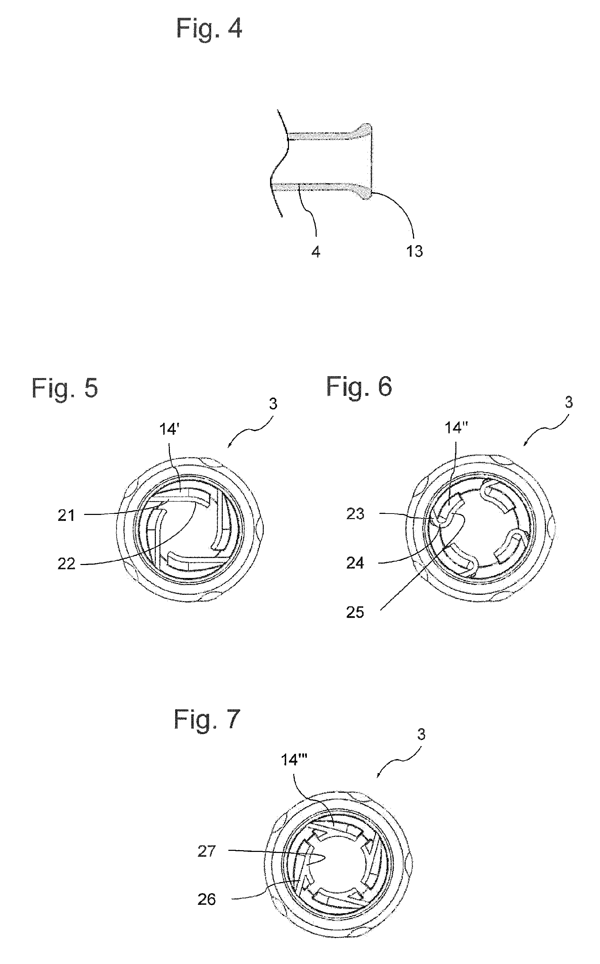

Figur 2 zeigt einen Ausschnitt aus Figur 1, jedoch ohne Tampon 5 und Rückzugsfaden 6 und in einem gegenüber Figur 1 vergrösserten Massstab. Am Innenumfang des Griffbereichs 10 sind Federlamellen 14 angeordnet, die in einem Fussbereich 15 mit der Wandung der Hülse 3 verbunden sind und deren freie Enden nach innen und in Richtung des stösselseitigen Endes der Hülse 3 orientiert sind. Die Kontaktflächen 17 der Federlamellen 14 mit dem Stössel 4 bilden einen Führungsbereich 18 für den Stössel 4. Im dargestellten Beispiel sind vier Federlamellen 14 am Innenumfang des Griffbereichs 10 angeordnet, wobei Figur 3 zeigt, dass jeweils zwischen zwei Federlamellen 14 eine Lücke 16 vorhanden ist, so dass sich die freien Enden der Federlamellen 14 federnd radial nach aussen und innen bewegen können. Im Aufnahmebereich 9 der Hülse 3 können nach innen oder aussen ragende Rippen 19 zur Erhöhung der Steifigkeit vorgesehen sein und im Griffbereich 10 kann eine Griffstruktur 20 angeordnet sein, um das Abrutschen der Finger beim Einführen der Anordnung 1 zu verhindern. Figur 4 zeigt einen Ausschnitt des Stössels 4 mit der Finger-Erweiterung 13 und macht deutlich, dass dieser Bereich eine erhöhte Wandstärke aufweisen kann, weil sich dieser Bereich bei der Montage des Stössels - im Gegensatz zum aus dem Dokument WO2012107880A2 bekannten Stand der Technik - nicht verformt.

Zur Montage des Stössels 4 in der Hülse 3 wird der Stössel 4 mit der Finger-Erweiterung 13 voran in das Einführungsende 7 der Hülse 3 eingeführt. Beim Passieren der Finger-Erweiterung 13 durch den Griffbereich 10 werden die freien Enden der Federlamellen 14 durch die Finger-Erweiterung 13 radial nach aussen gedrängt, federn nach dem Passieren der Finger-Erweiterung 13 zurück und liegen dann am Aussenumfang des Mittelbereichs des Stössels 4 an.

In einer nicht zeichnerisch dargestellten Ausführungsart können die freien Enden der Federlamellen 14 auch gegen das Einführungsende 7 der Hülse 3 gerichtet sein. Die Montage des Stössels erfolgt in diesem Fall vom stösselseitigen Ende 11 der Hülse 3 her, wobei der Stössel 4 mit der tamponseitigen Erweiterung 12 voran in das Innere des Griffbereichs 10 eingeführt wird.

Die Figuren 5 bis 7 zeigen weitere Ausführungsarten einer Hülse 3 des erfindungsgemässen Tamponapplikators 2, jeweils in einer Ansicht auf das stösselseitige Ende 11. Bei der Ausführungsart nach Figur 5 sind die Federlamellen 14' aus einem von der Wandung der Hülse 3 in Richtung einer Sehne ausgehenden geraden Bereich 21 und einem daran anschliessenden gebogenen Kontaktbereich 22 für den Stössel zusammengesetzt. Mit der Angabe "Richtung einer Sehne" ist gemeint, dass der genannte Bereich nicht zum Querschnittszentrum der Hülse 3 gerichtet ist, sondern etwa tangential zum im Führungsbereich 18 aufgenommenen Stössel 4. Auf diese Weise sind die Federlamellen 14', 14'', und 14''' fähig, während der Montage des Stössels beim Durchgang der Finger-Erweiterung 13 radial nach aussen federnd nachzugeben. Bei der Ausführungsart nach Figur 6 haben die Federlamellen 14'' ebenfalls einen geraden Bereich 23, der ebenfalls in Richtung einer Sehne von der Wand der Hülse 3 abragt, jedoch kürzer ist als der gerade Bereich 21 beim Ausführungsbeispiel nach Figur 5. An den geraden Bereich 23 schliesst ein Bogen 24 an, der seinerseits in einen Kontaktbereich 25 für den Stössel übergeht. Durch diese Gestaltung beanspruchen die Federlamellen 14'' in Umfangsrichtung weniger Platz als die Federlamellen 14' beim vorangehend beschriebenen Beispiel. Dies erlaubt es, beispielsweise fünf oder sechs Federlamellen 14'' am Innenumfang des Griffbereichs anzuordnen. Auch beim Ausführungsbeispiel gemäss Figur 7 sind wiederum von der Wand der Hülse 3 in Richtung einer Sehne ausgehende gerade Bereiche 26 vorhanden. Diese tragen an ihrem Ende Kontaktbereiche 27, die in Umfangsrichtung beiderseits vom geraden Bereich 26 abragen und so eine besonders gute Führung des Stössels 4 gewährleisten.

Bei den Ausführungsbeispielen der Figuren 5 bis 7 sind die Federlamellen 14', 14'' und 14''' derart angeordnet, dass ihre bei der Montage des Stössels 4 durch ein Ende des Stössels 4 zu kontaktierenden Bereiche dort eine grössere lichte Weite aufweisen, wo sie beim Einführen des Stössels 4 zuerst durch diesen kontaktiert werden. In den dargestellten Beispielen nimmt die durch die Kontaktbereiche 22, 25 und 27 begrenzte lichte Weite gegen das stösselseitige Ende 11 der Hülse 3 konusartig ab. Dem entsprechend wird der Stössel bei diesen Ausführungsbeispielen vom Einführungsende 7 der Hülse 3 her mit der Finger-Erweiterung 13 voran montiert. Bei nicht zeichnerisch dargestellten alternativen Beispielen könnte die Neigung der genannten Bereiche auch umgekehrt sein, so dass der Stössel 4 vom stösselseitigen Ende 11 der Hülse 3 her mit der tamponseitigen 12 Erweiterung voran montiert werden könnte.

Bei den Ausführungsbeispielen der Figuren 5 bis 7 sind die Kontaktbereiche 22, 25 und 27 nicht nur über die geraden Bereiche 21, 23 und 26 mit der Wandung der Hülse 3 verbunden, sondern zusätzlich auch an ihrer vom Betrachter abgewandten Seite, in ähnlicher Weise wie bei der Ausführungsart nach den Figuren 1 bis 3 durch den Fussbereich 15. Dies ist aber nicht zwingend, vielmehr kann jeder Kontaktbereich 22, 25 und 27 in Längsrichtung der Hülse 3 zwei freie Enden haben, nämlich sowohl wie dargestellt im Bereich des stösselseitigen Endes 11 als auch abweichend von den Darstellungen an ihrer vom stösselseitigen Ende 11 abgewandten Seite.

Bei allen zeichnerisch dargestellten und vorangehend beschriebenen Ausführungsbeispielen sind jeweils vier Federlamellen 14, 14', 14'' oder 14''' am Innenumfang im des Griffbereichs 10 verteilt angeordnet. Die Erfindung ist aber nicht auf vier Federlamellen beschränkt, sondern es können auch zwei, drei, fünf, sechs oder mehr Federlamellen vorhanden sein.

Bei allen zeichnerisch dargestellten und vorangehend beschriebenen Ausführungsbeispielen besteht die Hülse 3 vorzugsweise aus einem bevorzugt thermoplastischen Kunststoff und ich vorzugsweise im Spritzgussverfahren hergestellt. Bevorzugt sind dabei die Federlamellen 14, 14', 14'' oder 14''' einstückig mit der Hülse 3 geformt. Die Federlamellen 14, 14', 14'' oder 14''' können aber auch beispielsweise an einem Ring (nicht dargestellt) geformt sein, der mit der Hülse verbunden ist, beispielsweise über eine Schnappverbindung. Dadurch wird das Spektrum der Gestaltungsarten der Federlamellen werkzeugtechnisch erweitert, indem Federlamellen hergestellt werden können, die einstückig mit der Hülse nur schwer oder gar nicht realisierbar wären.

Der Ordnung halber sei abschliessend darauf hingewiesen, dass zum besseren Verständnis des Aufbaus der Tampon-Applikator-Anordnung diese beziehungsweise deren Komponenten teilweise unmassstäblich und/oder vergrössert und/oder verkleinert dargestellt sind.

**Bezugszeichenliste**

| | | | |
|---|---|---|---|
| 1 | Tampon-Applikator-Anordnung | 21 | gerader Bereich |
| | | 22 | Kontaktbereich |
| 2 | Tamponapplikator | 23 | gerader Bereich |
| 3 | Hülse | 24 | Bogen |
| 4 | Stössel | 25 | Kontaktbereich |
| 5 | Tampon | 26 | gerader Bereich |
| 6 | Rückzugsfaden | 27 | Kontaktbereich |
| 7 | Einführungsende | 28 | Längsachse |
| 8 | Lippen | | |
| 9 | Aufnahmebereich | | |
| 10 | Griffbereich | | |
| 11 | stösselseitiges Ende | | |
| 12 | tamponseitige Erweiterung | | |
| 13 | Finger-Erweiterung | | |
| 14 | Federlamelle | | |
| 14' | Federlamelle | | |
| 14'' | Federlamelle | | |
| 14''' | Federlamelle | | |
| 15 | Fussbereich | | |
| 16 | Lücke | | |
| 17 | Kontaktfläche | | |
| 18 | Führungsbereich | | |
| 19 | Rippe | | |
| 20 | Griffstruktur | | |

## Patentansprüche

1. Tamponapplikator (2) mit einer Hülse (3) zur Aufnahme eines Tampons (5) und einem Stössel (4) zum Ausstossen des Tampons (5) aus der Hülse (3), wobei die Hülse (3) ein Einführungsende (7) und ein stösselseitiges Ende (11) sowie im Bereich des stösselseitigen Endes einen Führungsbereich (18) für den Stössel (4) hat, wobei der Stössel (4) ein erstes, aufgeweitetes Ende (12) für den Kontakt mit dem Tampon (5) und ein zweites, aufgeweitetes Ende (13) für den Kontakt mit einem Finger hat und wobei der Innendurchmesser im Führungsbereich (18) der Hülse (3) kleiner ist als der Aussendurchmesser des ersten und zweiten Endes (12, 13) des Stössels (4), **dadurch gekennzeichnet, dass** der Führungsbereich (18) durch eine Anzahl am Innenumfang der Hülse (3) verteilt angeordnete, radial nachgiebige Federlamellen (14; 14'; 14''; 14''') gebildet ist.

2. Tamponapplikator (2) nach Anspruch 1, **dadurch gekennzeichnet, dass** die Hülse (3) einen Aufnahmebereich (9) für den Tampon (5) und einen im Bereich des stösselseitigen Endes (11) angeordneten Griffbereich (10) hat, dessen Aussendurchmesser kleiner ist als der Aussendurchmesser des Aufnahmebereichs (9).

3. Tamponapplikator (2) nach einem der vorangehenden Ansprüche, **dadurch gekennzeichnet, dass** der Stössel (4) hohl ist und dass das erste Ende (12) und/oder das zweite Ende (13) des Stössels (4) eine grössere Wandstärke aufweist als der zwischen den Enden (12, 13) liegende Teil des Stössels (4).

4. Tamponapplikator (2) nach einem der vorangehenden Ansprüche, **dadurch gekennzeichnet, dass** die Federlamellen (14) sich vom Innenumfang der Hülse (3) in Längsrichtung der Hülse erstrecken und zur Längsachse (28) der Hülse (3) hin geneigt sind.

5. Tamponapplikator nach Anspruch 4, **dadurch gekennzeichnet, dass** die Federlamellen (14) zum stösselseitigen Ende (11) der Hülse (3) hin geneigt sind.

6. Tamponapplikator nach Anspruch 4, **dadurch gekennzeichnet, dass** die Federlamellen (14) zum Einführungsende (7) der Hülse (3) hin geneigt sind.

7. Tamponapplikator (2) nach einem der vorangehenden Ansprüche, **dadurch gekennzeichnet, dass** die Federlamellen (14'; 14''; 14''') einen sich vom Innenumfang der Hülse (3) im Querschnitt gesehen in Richtung einer Sehne erstreckenden ersten Teil (21; 23; , 26) und daran anschliessend einen sich im Wesentlichen parallel zum Innenumfang der Hülse (3) erstreckenden zweiten Teil (22; 25; 27) aufweisen.

8. Tamponapplikator nach Anspruch 7, **dadurch gekennzeichnet, dass** die Federlamellen (14'') zwischen dem ersten Teil (23) und dem zweiten Teil (25) um mehr als 90 Grad umgebogen sind.

9. Tamponapplikator nach Anspruch 7, **dadurch gekennzeichnet, dass** der zweite Teil (27) der Federlamellen (14''') sich in Umfangsrichtung auf beiden Seiten des ersten Teils (26) erstreckt.

10. Verfahren zur Montage des Tamponapplikators (2) nach einem der vorangehenden Ansprüche, **dadurch gekennzeichnet, dass** der Stössel (4) montiert wird, indem er von einem Ende (7, 11) der Hülse (3) durch den Führungsbereich (18) geschoben wird, dass dabei das vorangehende Ende (12, 13) des Stössels (4) die Federlamellen (14; 14'; 14''; 14''') in einer im Wesentlichen radialen Richtung nach aussen drängt, so dass der Durchmesser des Führungsbereichs (18) vorübergehend aufgeweitet wird und dass nach dem Durchgang des vorangehenden Endes (12, 13) durch den Führungsbereich (18) die Federlamellen (14; 14'; 14''; 14''') ihre Position wieder einnehmen, die sie vor dem Durchgang des vorangehenden Endes (12, 13) des Stössels (4) hatten.
